# EUROPEAN PATENT APPLICATION

(11) **EP 4 368 186 A1**
(43) Date of publication of application: **15.05.2024**
(21) Application number: 22837759.4
(22) Date of filing: 08.07.2022
(51) Int. Cl.: A61K 31/70, A61K 31/7016, A61K 31/7088, A61K 31/712, A61K 31/7125, A61K 47/26, A61P 13/12, A61P 39/02, C12N 15/113

(54) **NEPHROTOXICITY REDUCING AGENT**

(30) Priority: 08.07.2021 JP 2021113563
(71) Applicant: Nippon Shinyaku Co., Ltd., Kyoto-shi Kyoto 601-8550 (JP)
(72) Inventor: HORIUCHI Takashi, Kyoto-shi, Kyoto 601-8550 (JP)
(74) Representative: J A Kemp LLP
(86) International application number: PCT/JP2022/027104
(87) International publication number: WO 2023/282345

(57) **Abstract**

In one embodiment, the object of the present invention is to provide a nephrotoxicity reducing agent for a pharmaceutical composition comprising an antisense oligomer, and a method for reducing nephrotoxicity of the pharmaceutical composition. In one embodiment, the present invention relates to a nephrotoxicity reducing agent for a pharmaceutical composition comprising an antisense oligomer, wherein the nephrotoxicity reducing agent comprises a non-glucose sugar and is used in an amount such that the concentration of the sugar in the pharmaceutical composition is 1 mg/mL to 400 mg/mL.

## Description

### Technical Field

The present invention relates to a nephrotoxicity reducing agent for a pharmaceutical composition comprising an antisense oligomer, a method for reducing nephrotoxicity of the pharmaceutical composition, a pharmaceutical composition comprising an antisense oligomer with reduced nephrotoxicity, and the like.

### Background Art

Antisense oligomers are nucleic acids that sequence-specifically hybridize to target mRNAs and pre-mRNAs. The antisense oligomers exert their actions by degradation of mRNAs and pre-mRNAs, exon-skipping, exon-inclusion, translation inhibition and the like, and thus have been used as therapeutic agents for some diseases. For example, Japanese Patent Laid-Open No. 2015-91229 (Patent Literature 1) discloses an antisense nucleic acid which can treat Fukuyama muscular dystrophy by normalizing aberrant splicing of a fukutin gene having an insertion mutation of SVA retrotransposon.

However, with respect to the antisense oligomers, it has been known that, for example, morpholino oligomers are accumulated in the kidney (Non Patent Literature 1, Figure 2), administration of morpholino oligomers causes nephrotoxicity to be expressed (Non Patent Literature 2, Figure 3, Table 5), and the administration of morpholino oligomers induces basophilic substances in the kidney tubule (Non Patent Literature 3, Figure 1). A method for avoiding the appearance of the nephrotoxicity and the basophilic substances in the renal tubule has not been established. On the other hand, it has been known the following: for oligonucleotides that have greater toxicity in the aggregate form than in monomeric form, heating with use of chemical species such as mannitol that disrupt aggregates can suppress multimerization in formulations of therapeutic oligonucleotides (Patent Literature 2); and saccharides and sugar alcohols in liquid formulations suppress aggregation of oligonucleotides (Patent Literature 3).

### Citation List

### Patent Literature

Patent Literature 1: Japanese Patent Laid-Open No. 2015-91229
Patent Literature 2: International Publication No. 2002/036767
Patent Literature 3: International Publication No. 2010/009038

### Non Patent Literature

Non Patent Literature 1: Heemskerk HA, de Winter CL, de Kimpe SJ, van Kuik-Romeijn P, Heuvelmans N, Platenburg GJ, van Ommen GJ, van Deutekom JC, Aartsma-Rus A. In vivo comparison of 2'-O-methyl phosphorothioate and morpholino antisense oligonucleotides for Duchenne muscular dystrophy exon skipping. J Gene Med. 2009 Mar; 11(3):257-66. doi: 10.1002/jgm.1288.
Non Patent Literature 2: Sazani P, Ness KP, Weller DL, Poage D, Nelson K, Shrewsbury AS. Chemical and mechanistic toxicology evaluation of exon skipping phosphorodiamidate morpholino oligomers in mdx mice. Int J Toxicol. 2011 May; 30(3):322-33. doi: 10.1177/1091581811403504. Epub 2011 May 3.
Non Patent Literature 3: Carver MP, Charleston JS, Shanks C, Zhang J, Mense M, Sharma AK, Kaur H, Sazani P. Toxicological Characterization of Exon Skipping Phosphorodiamidate Morpholino Oligomers (PMOs) in Non-human Primates. J Neuromuscul Dis. 2016 Aug 30; 3(3):381-393. doi: 10.3233/JND-160157.

### Summary of Invention

### Technical Problem

It has conventionally been known that pharmaceutical compositions comprising antisense oligomers have a nephrotoxicity problem. In such a circumstance, it is desirable to provide an improved pharmaceutical composition comprising antisense oligomers.

### Solution to Problem

That is, the present invention provides a nephrotoxicity reducing agent for a pharmaceutical composition comprising an antisense oligomer, a method for reducing nephrotoxicity of the pharmaceutical composition, a pharmaceutical composition comprising an antisense oligomer with reduced nephrotoxicity, and the like as follows.
(1) A nephrotoxicity reducing agent for a pharmaceutical composition comprising an antisense oligomer, wherein the nephrotoxicity reducing agent comprises a non-glucose sugar and is used in an amount such that the concentration of the sugar in the pharmaceutical composition is 1 mg/mL to 400 mg/mL.
(2) The nephrotoxicity reducing agent according to (1), wherein the nephrotoxicity reducing agent is used in an amount such that the concentration of the non-glucose sugar in the pharmaceutical composition is 5 mg/mL to 340 mg/mL.
(3) The nephrotoxicity reducing agent according to (1) or (2), wherein the concentration of the antisense oligomer in the pharmaceutical composition is 0.5 mg/mL to 200 mg/mL.
(4-1) A nephrotoxicity reducing agent for a pharmaceutical composition comprising an antisense oligomer, wherein the nephrotoxicity reducing agent comprises a non-glucose sugar and is used in an amount such that the weight ratio of the sugar is 0.05 to 30 per 1 of the antisense oligomer.
(4-2) The nephrotoxicity reducing agent according to (4-1), wherein the pharmaceutical composition and the nephrotoxicity reducing agent are administered separately.
(5) The nephrotoxicity reducing agent according to (4-1) or (4-2), wherein the nephrotoxicity reducing agent is used in an amount such that the weight ratio of the sugar is 0.1 to 13.3 per 1 of the antisense oligomer.
(6) The nephrotoxicity reducing agent according to any of (1) to (5), wherein the sugar is a disaccharide.
(7) The nephrotoxicity reducing agent according to any of (1) to (6), wherein the sugar is sucrose.
(8) The nephrotoxicity reducing agent according to any of (1) to (6), wherein the sugar is trehalose.
(9) The nephrotoxicity reducing agent according to any of (1) to (8), wherein the antisense oligomer is a morpholino oligomer.
(10) The nephrotoxicity reducing agent according to any of (1) to (9), wherein the antisense oligomer is a phosphorodiamidate morpholino oligomer.
(11) The nephrotoxicity reducing agent according to any of (1) to (10), wherein the 5'-end of the antisense oligomer is any group represented by the following chemical formulae (1) and (2): 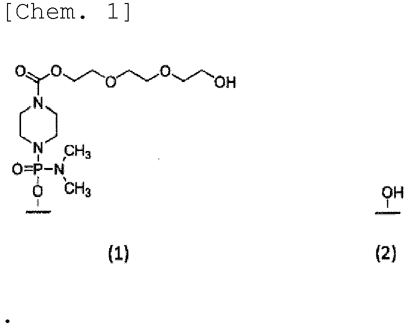
(12) The nephrotoxicity reducing agent according to any of (1) to (11), wherein the antisense oligomer comprises four consecutive purine bases in its base sequence.
(13) The nephrotoxicity reducing agent according to (12), wherein at least two of the four consecutive purine bases are guanine.
(14) The nephrotoxicity reducing agent according to any of (1) to (13), wherein the antisense oligomer comprises a base sequence selected from the group consisting of SEQ ID NOs: 1 to 12.
(15-1) A method for reducing nephrotoxicity for a pharmaceutical composition comprising an antisense oligomer, comprising adding a non-glucose sugar in an amount such that the concentration of the sugar in the pharmaceutical composition is 1 mg/mL to 400 mg/mL.
(15-2) The method according to (15-1), wherein the non-glucose sugar is added in an amount such that the concentration of the sugar in the pharmaceutical composition is 5 mg/mL to 340 mg/mL.
(15-3) The method according to (15-1) or (15-2), wherein the concentration of the antisense oligomer in the pharmaceutical composition is 0.5 mg/mL to 200 mg/mL.
(16-1) A method for reducing nephrotoxicity of an antisense oligomer in a subject to whom the antisense oligomer has been administered, comprising administering a non-glucose sugar to the subject, wherein the sugar is administered in an amount such that the weight ratio of the sugar is 0.05 to 30 per 1 of the antisense oligomer.
(16-2) The method according to (16-1), wherein the antisense oligomer and the sugar are administered separately.
(16-3) The method according to (16-1) or (16-2), wherein the sugar is used in an amount such that the weight ratio of the sugar is 0.1 to 13.3 per 1 of the antisense oligomer.
(17-1) A pharmaceutical composition comprising an antisense oligomer with reduced nephrotoxicity, wherein the pharmaceutical composition comprises a nephrotoxicity reducing agent comprising a non-glucose sugar at a concentration of 1 mg/mL to 400 mg/mL.
(17-2) The pharmaceutical composition according to (17-1), wherein the pharmaceutical composition comprises the nephrotoxicity reducing agent comprising the non-glucose sugar at a concentration of 5 mg/mL to 340 mg/mL.
(17-3) The pharmaceutical composition according to (17-1) or (17-2), wherein the concentration of the antisense oligomer in the pharmaceutical composition is 0.5 mg/mL to 200 mg/mL.
(18) The method or pharmaceutical composition according to any of (15-1) to (17-3), wherein the sugar is a disaccharide.
(19) The method or pharmaceutical composition according to any of (15-1) to (18), wherein the sugar is sucrose.
(20) The method or pharmaceutical composition according to any of (15-1) to (18), wherein the sugar is trehalose.
(21) The method or pharmaceutical composition according to any of (15-1) to (20), wherein the antisense oligomer is a morpholino oligomer.
(22) The method or pharmaceutical composition according to any of (15-1) to (21), wherein the antisense oligomer is a phosphorodiamidate morpholino oligomer.
(23) The method or pharmaceutical composition according to any of (15-1) to (22), wherein the 5'-end of the antisense oligomer is any group represented by the following chemical formulae (1) and (2): 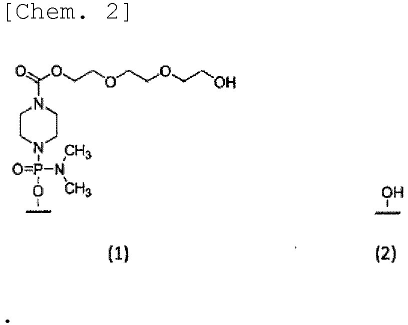
(24) The method or pharmaceutical composition according to any of (15-1) to (23), wherein the antisense oligomer comprises four consecutive purine bases in its base sequence.
(25) The method or pharmaceutical composition according to (24), wherein at least two of the four consecutive purine bases are guanine.
(26) The method or pharmaceutical composition according to any of (15-1) to (24), wherein the antisense oligomer comprises a base sequence selected from the group consisting of SEQ ID NOs: 1 to 12.

### Advantageous Effect of Invention

The present invention provides a nephrotoxicity reducing agent for a pharmaceutical composition comprising an antisense oligomer, and a method for reducing nephrotoxicity of the pharmaceutical composition.

### Brief Description of Drawings

[Figure 1] Figure 1 shows a hematoxylin-eosin staining image of a kidney when a PMO No. 8 dosing solution (50 mg/mL of PMO No. 8) is administered at 10 mL/kg.
[Figure 2] Figure 2 shows a hematoxylin-eosin staining image of a kidney when a PMO No. 8 dosing solution (50 mg/mL of PMO No. 8, and 20 mg/mL of sucrose) is administered at 10 mL/kg.
[Figure 3] Figure 3 shows a hematoxylin-eosin staining image of a kidney when a PMO No. 8 dosing solution (50 mg/mL of PMO No. 8, and 50 mg/mL of sucrose) is administered at 10 mL/kg.
[Figure 4] Figure 4 shows a hematoxylin-eosin staining image of a kidney when a PMO No. 8 dosing solution (50 mg/mL of PMO No. 8, and 100 mg/mL of sucrose) is administered at 10 mL/kg.
[Figure 5] Figure 5 shows absorbance measurement values at 620 nm under the conditions shown in Table 6.
[Figure 6] Figure 6 shows absorbance measurement values at 620 nm under the conditions shown in Table 7.
[Figure 7] Figure 7 shows absorbance measurement values at 620 nm under the conditions shown in Table 8.
[Figure 8] Figure 8 shows absorbance measurement values at 620 nm under the conditions shown in Table 9.
[Figure 9] Figure 9 shows absorbance measurement values at 620 nm under the conditions shown in Table 10.
[Figure 10] Figure 10 shows absorbance measurement values at 620 nm under the conditions shown in Table 11.
[Figure 11] Figure 11 shows absorbance measurement values at 620 nm under the conditions shown in Table 12.
[Figure 12] Figure 12 shows absorbance measurement values at 620 nm under the conditions shown in Table 13.
[Figure 13] Figure 13 shows absorbance measurement values at 620 nm under the conditions shown in Table 18.
[Figure 14] Figure 14 shows absorbance measurement values at 620 nm under the conditions shown in Table 19.
[Figure 15] Figure 15 shows absorbance measurement values at 620 nm under the conditions shown in Table 20.
[Figure 16] Figure 16 shows absorbance measurement values at 620 nm under the conditions shown in Table 21.
[Figure 17] Figure 17 shows absorbance measurement values at 620 nm under the conditions shown in Table 22.
[Figure 18] Figure 18 shows absorbance measurement values at 620 nm under the conditions shown in Table 23.
[Figure 19] Figure 19 shows absorbance measurement values at 620 nm under the conditions shown in Table 24.

### Description of Embodiments

In one embodiment, the present invention relates to a nephrotoxicity reducing agent for a pharmaceutical composition comprising an antisense oligomer. The term "nephrotoxicity reducing agent" as used herein means an agent for reducing nephrotoxicity or an event that causes the nephrotoxicity (e.g., accumulation of basophilic substances) that a pharmaceutical composition comprising an antisense oligomer may have. Distribution and accumulation of administered antisense oligomers at a high concentration in the kidney during the excretion process may cause basophilic substances to be observed in the kidney and/or lumens of kidney tubules. The term nephrotoxicity means occurrence of tissue damage or a decrease in kidney function due to higher accumulation of the antisense oligomers in the kidney. The tissue damage in the kidney is known to cause, for example, expansion and necrosis of renal tubule, and the decrease in kidney function is widely known to cause an increase in blood urea nitrogen (BUN) values and blood creatinine (Cre) values, for example. By reducing the nephrotoxicity of the antisense oligomers, a pharmaceutical composition comprising a high dose of antisense oligomers, and a method for treating diseases using the pharmaceutical composition can be provided.

The presence or absence of nephrotoxicity reducing effects can be determined, for example, by measuring the blood urea nitrogen (BUN) values and blood creatinine (Cre) values with, for example, a urease-GIDH method and an enzymatic method, respectively, as described in Examples. For example, it can be determined to be effective in reducing nephrotoxicity when the BUN values and/or Cre values are reduced, for example, by 5% or more, 100 or more, 20% or more, 30% or more, 40% or more, or 50% or more in the case of administering the nephrotoxicity reducing agent, compared to a case of administering no nephrotoxicity reducing agent.

In one embodiment, the nephrotoxicity reducing agent of the present invention may consist of or comprise a sugar (except glucose). The sugar may be a sugar of two or more saccharides or may be a disaccharide. Examples of the sugar include, but not limited to, disaccharides such as sucrose, lactose, lactulose, trehalose, maltose, and isomaltose; trisaccharide's such as raffinose, melezitose, and maltotriose; and monosaccharides such as galactose, mannose, fructose, ribose, xylose, arabinose, and lyxose. In one embodiment, the sugar is or comprises sucrose. In another embodiment, the sugar is or comprises trehalose.

When comprising an ingredient other than the sugar, the nephrotoxicity reducing agent of the present invention can be formulated by appropriately blending a pharmaceutically acceptable carrier or additive (and optionally the nephrotoxicity reducing agent of the present invention) to the sugar. Specifically, it can be formulated into oral agents such as tablets, coated tablets, pills, powders, granules, capsules, liquids, suspensions, and emulsions; and parenteral agents such as injectables, infusions, suppositories, ointments and patches. The parenteral agents are preferable. The injectables may be lyophilized preparations. The blending proportion of the carrier or additive can be appropriately set according to the range usually employed in the pharmaceutical field. Examples of the carriers or additives to be blended include, but not particularly limited, various carriers such as water, physiological saline, other aqueous solvents, and aqueous or oily bases, and various additives such as excipients, binders, pH adjusters, disintegrants, absorption promoters, lubricants, colorants, corrigents, and flavors.

The additives miscible with tablets, capsules or the like are used that include, for example, binders such as gelatin, corn starch, tragacanth, or gum arabic, excipients such as crystalline cellulose, swelling agents such as corn starch, gelatin, or alginic acid, lubricants such as magnesium stearate, sweetening agents such as sucrose, lactose, or saccharin, flavoring agents such as peppermint, Gaultheria adenothrix oil, or cherries. When the formulated dosage unit form is a capsule, a liquid carrier such as oil/fat can be further comprised in the materials of the above types. A sterile composition for injection can be prepared according to the normal pharmaceutical practice (e.g., dissolution or suspension of active ingredients into solvents such as water for injection, or natural vegetable oil). As the aqueous solutions for injection, isotonic solutions (e.g., sodium chloride) comprising physiological saline, glucose, or other aid agents are used and can be combined with an adequate solubilizer such as alcohol (e.g., ethanol), polyalcohol (e.g., propylene glycol or polyethylene glycol), and a nonionic surfactant (e.g., polysorbate 80 (TM) or HCO-50). As the oily solutions, for example, sesame oil or soybean oil is used and can be combined with a solubilizer, such as benzyl benzoate or benzyl alcohol. In addition, buffers (e.g., phosphate buffer solution or sodium acetate buffer solution), soothing agents (e.g., benzalkonium chloride or procaine hydrochloride), stabilizers (e.g., human serum albumin or polyethylene glycol), preservatives (e.g., benzyl alcohol or phenol), or antioxidants may be blended therewith. Further, it can be lyophilized preparations.

As used herein, the antisense oligomer may be any of an oligonucleotide, a morpholino oligomer, or a peptide nucleic acid (PNA) oligomer (hereinafter, also referred to as "antisense oligonucleotide described herein", "antisense morpholino oligomer described herein", "antisense peptide nucleic acid oligomer described herein", respectively).

The antisense oligonucleotide is an antisense oligomer whose constituent unit is a nucleotide, and the nucleotide may be any of a ribonucleotide, a deoxyribonucleotide, or a modified nucleotide.

The modified nucleotide refers to those in which all or part of nucleobase, sugar moiety and phosphate-binding moiety constituting ribonucleotides or deoxyribonucleotides are modified.

Examples of the nucleobases can include adenine, guanine, hypoxanthine, cytosine, thymine, uracil, or modified bases thereof. Examples of the modified bases include, but not limited to, pseudouracil, 3-methyluracil, dihydrouracil, 5-alkynecytosine (e.g., 5-methylcytosine), 5-alkyluracil (e.g., 5-ethyluracil), 5-halouracil (e.g., 5-bromouracil), 6-azapyrimidine, 6-alkylpyrimidine (e.g., 6-methyluracil), 2-thiouracil, 4-thiouracil, 4-acetylcytosine, 5-(carboxyhydroxymethyl)uracil, 5-carboxymethylaminomethyl-2-thiouracil, 5-carboxymethylaminomethyl uracil, 1-methyladenine, 1-methylhypoxanthine, 2,2-dimethylguanine, 3-methylcytosine, 2-methyladenine, 2-methylguanine, N6-methyladenine, 7-methylguanine, 5-methoxyaminomethyl-2-thiouracil, 5-methylaminomethyluracil, 5-methylcarbonylmethyluracil, 5-methyloxyuracil, 5-methyl-2-thiouracil, 2-methylthio-N6-isopentenyladenine, uracil-5-oxyacetic acid, 2-thiocytosine, purine, 2,6-diaminopurine, 2-aminopurine, isoguanine, indole, imidazole, and xanthine.

Examples of the modification of the sugar moiety can include modification at the 2' position of ribose, and modification of other parts of the sugar. Examples of the modification at the 2' position of ribose can include modification wherein -OH group at the 2' position of ribose is substituted with -OR, -R, -R'OR, -SH, -SR, -NH₂, -NHR, -NR₂, -N₃, -CN, -F, -Cl, -Br, and -I. Here, R represents alkyl or aryl. R' represents alkylene.

Examples of the modification of other parts of the sugar include, but not limited to, those in which O at the 4' position of ribose or deoxyribose is substituted with S, and the 2' and 4' positions of the sugar are cross-linked, for example, a locked nucleic acid (LNA) or 2'-O,4'-C-ethylene-bridged nucleic acid (ENA).

Examples of the modification of phosphate-binding moiety can include modifications wherein the phosphodiester bond is substituted with a phosphorothioate bond, a phosphorodithioate bond, an alkylphosphonate bond, a phosphoramidate bond, and a boranophosphate bond (see, e.g., Enya et al: Bioorganic & Medicinal Chemistry, 2008, 18, 9154-9160) (see, e.g., Japanese Re-Publication of International Patent Application Nos. 2006/129594 and 2006/038608).

As used herein, the alkyl is preferably a linear or branched alkyl having 1 to 6 carbon atoms. Specific examples thereof include methyl, ethyl, n-propyl, isopropyl, n-butyl, isobutyl, sec-butyl, tert-butyl, n-pentyl, isopentyl, neopentyl, tert-pentyl, n-hexyl, and isohexyl. The alkyl may be substituted, examples of the substituent therefor can include halogen, alkoxy, cyano, and nitro, and 1 to 3 positions may be substituted with these substituents.

As used herein, the cycloalkyl is preferably those having 3 to 12 carbon atoms. Specific examples thereof include cyclopropyl, cyclobutyl, cyclopentyl, cyclohexyl, cycloheptyl, cyclooctyl, cyclodecyl, and cyclododecyl.

As used herein, examples of the halogen can include fluorine, chlorine, bromine, and iodine.

As used herein, examples of the alkoxy include a linear or branched alkoxy having 1 to 6 carbon atoms, and for example, methoxy, ethoxy, n-propoxy, isopropoxy, n-butoxy, isobutoxy, sec-butoxy, tert-butoxy, n-pentyloxy, isopentyloxy, n-hexyloxy, and isohexyloxy. Alkoxy having 1 to 3 carbon atoms is preferable among others.

As used herein, the aryl is preferably those having 6 to 10 carbon atoms. Specific examples thereof can include phenyl, α-naphthyl, and β-naphthyl. Phenyl is preferable among others. The aryl may be substituted, examples of the substituent therefor can include alkyl, halogen, alkoxy, cyano, and nitro, and the aryl may be substituted with 1 to 3 of these substituents.

As used herein, the alkylene is preferably a linear or branched alkylene having 1 to 6 carbon atoms. Specific examples thereof can include methylene, ethylene, trimethylene, tetramethylene, pentamethylene, hexamethylene, 2-(ethyl)trimethylene, and 1-(methyl)tetramethylene.

As used herein, examples of acyl can include a linear or branched alkanoyl or aroyl. Examples of the alkanoyl can include formyl, acetyl, 2-methylacetyl, 2,2-dimethylacetyl, propionyl, butyryl, isobutyryl, pentanoyl, 2,2-dimethylpropionyl, and hexanoyl. Examples of the aroyl can include benzoyl, toluoyl, and naphthoyl. The aroyl may be substituted at a substitutable position, or may be substituted with alkyl.

The antisense oligonucleotide described herein can be easily synthesized with various automated synthesizers (e.g., AKTA oligopilot plus 10 / 100 (GE Healthcare)). Alternatively, it can be made by entrusting any third party institution (e.g., Promega Corporation or Takara Bio Inc.).

The antisense morpholino oligomer described herein is an antisense oligomer whose constituent unit is a group represented by the following general formula: 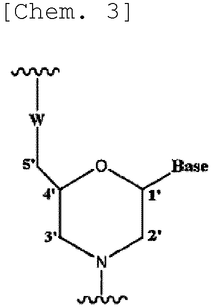 wherein, Base represents a nucleobase; and
W represents a group represented by any of the following formulae: 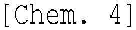 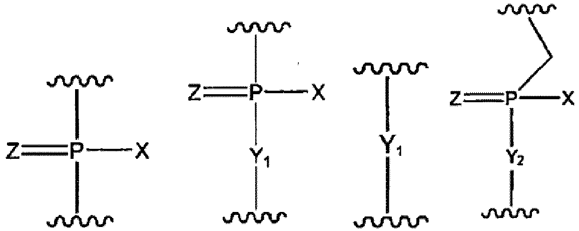 wherein, X represents -CH₂R¹, -O-CH₂R¹, -S-CH₂R¹, -NR²R³, or F;
R¹ represents H, or alkyl;
R² and R³ are the same or different, and represent H, alkyl, cycloalkyl, or aryl;
Y₁ represents O, S, CH₂, or NR¹;
Y₂ represents O, S, or NR¹; and
Z represents O or S.

As used herein, the morpholino oligomer is preferably an oligomer whose constituent unit is a group represented by the following formula (phosphorodiamidate morpholino oligomer, hereinafter referred to as "PMO"): 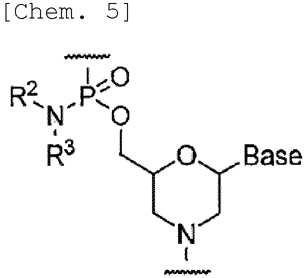 wherein, Base, R² and R³ are as defined above.

The morpholino oligomer can be produced according to the methods described in International Publication Nos. 1991/009033, or 2009/064471, for example. Particularly, PMO can be produced according to the methods described in International Publication Nos. 2009/064471, or 2013/100190.

The antisense peptide nucleic acid oligomer described herein is an antisense oligomer whose constituent unit is a group represented by the following general formula: 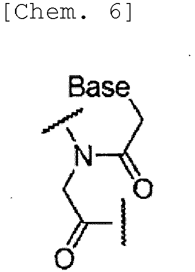 wherein, Base is as defined above.

The peptide nucleic acid oligomer can be produced, for example, according to the following references:
1) P. E. Nielsen, M. Egholm, R. H. Berg, O. Buchardt, Science, 254, 1497 (1991);
2) M. Egholm, O. Buchardt, P. E. Nielsen, R. H. Berg, JACS, 114, 1895 (1992);
3) K. L. Dueholm, M. Egholm, C. Behrens, L. Christensen, H. F. Hansen, T. Vulpius, K. H. Petersen, R. H. Berg, P. E. Nielsen, O. Buchardt, J. Org. Chem., 59, 5767 (1994);
4) L. Christensen, R. Fitzpatrick, B. Gildea, K. H. Petersen, H. F. Hansen, T. Koch, M. Egholm, O. Buchardt, P. E. Nielsen, J. Coull, R. H. Berg, J. Pept. Sci., 1, 175 (1995); and
5) T. Koch, H. F. Hansen, P. Andersen, T. Larsen, H. G. Batz, K. Otteson, H. Orum, J. Pept. Res., 49, 80 (1997).

The antisense oligomer described herein may be in the form of a pharmaceutically acceptable salt, in the form of a hydrate, or in the form of a hydrate of a pharmaceutically acceptable salt thereof.

Examples of the pharmaceutically acceptable salt of the antisense oligomer described herein include alkali metal salts such as a sodium salt, a potassium salt, and a lithium salt; alkaline earth metal salts such as a calcium salt, and a magnesium salt; metal salts such as an aluminum salt, an iron salt, a zinc salt, a copper salt, a nickel salt, and a cobalt salt; an ammonium salt; organic amine salts such as a t-octylamine salt, a dibenzylamine salt, a morpholine salt, a glucosamine salt, a phenylglycine alkyl ester salt, an ethylenediamine salt, an N-methylglucamine salt, a guanidine salt, a diethylamine salt, a triethylamine salt, a dicyclohexylamine salt, an N,N'-dibenzylethylenediamine salt, a chloroprocaine salt, a procaine salt, a diethanolamine salt, an N-benzyl-phenethylamine salt, a piperazine salt, a tetramethylammonium salt, and a tris(hydroxymethyl)aminomethane salt; hydrohalide salts such as a hydrofluoride salt, a hydrochloride salt, a hydrobromide salt, and a hydroiodide salt; inorganic acid salts such as a nitrate salt, a perchlorate salt, a sulfate salt, and a phosphate salt; lower alkanesulfonate salts such as a methanesulfonate salt, a trifluoromethanesulfonate salt, and an ethanesulfonate salt; arylsulfonate salts such as a benzenesulfonate salt, and p-toluenesulfonate salt; organic acid salts such as an acetate salt, a malate salt, a fumarate salt, a succinate salt, a citrate salt, a tartrate salt, an oxalate salt, and a maleate salt; and amino acid salts such as a glycine salt, a lysine salt, an arginine salt, an ornithine salt, a glutamate salt, an aspartate salt. These salts can be produced by a known method. Alternatively the antisense oligomer described herein may be in the form of a hydrate thereof.

The antisense oligomer described herein may have any of the groups represented in the following chemical formulae (1) to (3) at its 5'-end. Preferably, it is either group (1) or (2).

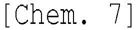

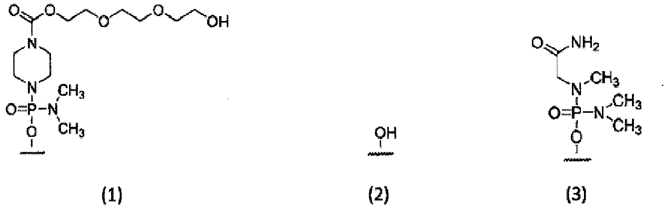

Hereinafter, the groups represented by (1), (2), and (3) above are referred to as "group (1)", "group (2)", and "group (3)", respectively.

The base sequence of the antisense oligomer described herein is not limited, and for example, the antisense oligomer may comprise four consecutive purine bases in its base sequence. Further, at least two of the four consecutive purine bases may be guanine.

In one embodiment, the antisense oligomer described herein comprises or consists of (i) a base sequence selected from the group consisting of SEQ ID NO: 1 to 12, (ii) a base sequence having a sequence identity of 80% or more, 85% or more, 86% or more, 87% or more, 88% or more, 89% or more, 90% or more, 910 or more, 92% or more, 93% or more, 94% or more, 95% or more, 96% or more, 97% or more, 98% or more, or 99% or more to the base sequence selected from the group consisting of SEQ ID NO: 1 to 12, or (iii) a basic sequence wherein one or several bases are added, deleted or substituted in the base sequence selected from the group consisting of SEQ ID NO: 1 to 12.

The term "identity" of the base sequences as used herein means a proportion of matched bases when two base sequences are aligned with each other. The sequence identity can be determined by using FASTA (Science 227 (4693): 1435-1441, (1985)) or the algorithm of Karlin and Altschul, BLAST (Basic Local Alignment Search Tool) (Proc. Natl. Acad. Sci. USA 872264-2268, 1990; Proc Natl Acad Sci USA 90: 5873, 1993). Programs based on the algorithm of BLAST and referred to as blastn, blastx, tblastn, and tblastx have been developed (Altschul SF, et al: J Mol Biol 215: 403, 1990). When blastn is used for base sequence analysis, parameters are set to, for example, score = 100, and wordlength = 12. When BLAST and Gapped BLAST programs are used, default parameters for each program are used.

As used herein, the term "several" in the base sequence wherein one or several bases are added, deleted, or substituted means 2, 3, 4, 5, 6, 7, 8, 9, or 10.

In one embodiment, the antisense oligomer described herein is one that targets a sequence ranging from positions 115937 to 115981 of a genome sequence (SEQ ID NO: 13) of the insertion mutant of the fukutin gene wherein an SVA retrotransposon sequence (GenBank ACCESSION: AB185332) is inserted into the genome sequence of the fukutin gene (GenBank ACCESSION: AB038490) or one that does not target the sequence of the above range. Examples of the antisense oligomer that targets a sequence ranging from positions 115937 to 115981 of SEQ ID NO: 13 include antisense oligomers comprising a base sequence selected from the group consisting of SEQ ID NO: 1 to 7. Examples of the antisense oligomer that does not target a sequence ranging from positions 115937 to 115981 of SEQ ID NO: 13 include antisense oligomers comprising a base sequence selected from the group consisting of SEQ ID NO: 8 to 12.

In one embodiment, the antisense oligomer described herein is a conjugate to which a functional peptide, for example, cell-permeable peptide (CPP) is attached. Known functional peptides or commercially-available functional peptides can be used herein. Examples of the functional peptides that can be used herein include an arginine-rich peptide disclosed in International Publication No. 2008/036127; a peptide targeting organs disclosed in International Publication No. 2009/005793, for example, RXR, or RBR; and a peptide comprising amino acid subunits disclosed in International Publication No. 2012/150960. The cell-permeable peptide (CPP) can pass through cell membranes of mammalian cells, and accordingly, it represents a short peptide sequence having 10 to about 30 amino acids capable of improving cell drug delivery (see, e.g., Hum Mol Genet. 2011 Aug 15; 20(16): 3151-3160; Pharmacology & Therapeutics 154 (2015) 78-86). Known CPPs or commercially-available CPPs can be used herein. Examples of the CPPs that can be used herein include, the CPPs listed in Pharmacology & Therapeutics 154 (2015) 78-86, p.80, Table 1, such as TAT(48-60), penetratin, polyarginine, Oct4, WT1-pTj, DPV3, transportan, MAP, VP22, Rep1, KW, KFGF, FGF12, integrin β3 peptide, C105Y, TP2; and CPPs listed in Japanese Translation of PCT International Application Publication No. 2017-500856 (International Publication No. 2015/089487), paragraph [0085], Table 1, such as DPV10/6, DPV15b, YM-3, Tat, LR11, C45D18, Lyp-1, Lyp-2, BMV GAG, hLF1-22, C45D18, LR20. The CPPs are commercially available from Funakoshi, Co., Ltd., for example. Commercially available CPPs such as TAT (Funakoshi, Co., Ltd.), penetratin (Funakoshi, Co., Ltd.), or known CPPs such as R8 can be used herein. Examples of preferable CPPs that can be used herein include hLIMK, TAT, penetratin, and R8 (see, e.g., International Publication Nos. 2016/187425, 2018/118662, 2018/118599, and 2018/118627, and EBioMedicine 45 (2019) 630-645). The CPP can be directly bound to the antisense oligomer described herein, or can be bound via a linker capable of binding the CPP to the antisense oligomer. Known linkers can be used herein. Examples of the linker include those described in Japanese Translation of PCT International Application Publication No. 2017-500856 (International Publication No. 2015/089487), International Publication Nos. 2015/089487, 2009-073809, 2013/075035, 2015/105083, 2014/179620, 2015/006740, and 2017/010575. Examples of preferable linkers that can be used herein include 4-maleimidobutyrate, a linker capable of binding to the functional peptide or antisense oligomer described herein via disulfide bond. The conjugate as used herein can be prepared by a method known to those skilled in the art.

The pharmaceutical composition described herein may be formulated by appropriately blending a pharmaceutically acceptable carrier or additive (and optionally the sugar of the present invention). The pharmaceutically acceptable carrier or additive and formulation for the pharmaceutical composition is the same as those described for the nephrotoxicity reducing agent of the present invention except that the antisense nucleic acid is the active ingredient.

In one embodiment, the nephrotoxicity reducing agent of the present invention is used and/or added to the pharmaceutical composition in an amount such that the concentration of the sugar in the pharmaceutical composition described herein is 1 mg/mL to 400 mg/mL, for example, 5 mg/mL to 340 mg/mL. In one embodiment, the nephrotoxicity reducing agent of the present invention is used and/or added to the pharmaceutical composition in an amount such that the concentration of the sugar in the pharmaceutical composition is 1 mg/mL or more, 2.5 mg/mL or more, 3 mg/mL or more, 4 mg/mL or more, 5 mg/mL or more, 10 mg/mL or more, 15 mg/mL or more, 20 mg/mL or more, 30 mg/mL or more, or 40 mg/mL or more. Further, the nephrotoxicity reducing agent of the present invention is used and/or added to the pharmaceutical composition in an amount such that the concentration of the sugar in the pharmaceutical composition is 400 mg/mL or less, 350 mg/mL or less, 340 mg/mL or less, 335 mg/mL or less, 330 mg/mL or less, 300 mg/mL or less, 250 mg/mL or less, or 200 mg/mL or less, for example.

In one embodiment, the concentration of the antisense oligomer in the pharmaceutical composition described herein is 0.5 mg/mL to 200 mg/mL, for example, 16 mg/mL to 130 mg/mL. In one embodiment, the concentration of the antisense oligomer in the pharmaceutical composition described herein may be 0.5 mg/mL or more, 1 mg/mL or more, 2 mg/mL or more, 3 mg/mL or more, 4 mg/mL or more, 5 mg/mL or more, 10 mg/mL or more, or 16 mg/mL or more, and may be 200 mg/mL or less, 180 mg/mL or less, 150 mg/mL or less, 140 mg/mL or less, or 130 mg/mL or less.

In one embodiment, the present invention relates to a nephrotoxicity reducing agent for a pharmaceutical composition comprising an antisense oligomer, wherein the nephrotoxicity reducing agent comprises a sugar and is used in an amount such that the weight ratio of the sugar is 0.05 to 30, for example, 0.1 to 13.3 per 1 of the antisense oligomer. The pharmaceutical composition comprising an antisense oligomer and the sugar are as described above. In one embodiment, the nephrotoxicity reducing agent of the present invention is used in an amount such that the weight ratio of the sugar is 0.05 or more, 0.1 or more, 0.15 or more, 0.2 or more, 0.3 or more, 0.4 or more, 0.5 or more, 0.6 or more, 0.7 or more, 0.8 or more, 0.9 or more, 1 or more, 2 or more, or 5 or more per 1 of the antisense oligomer. In one embodiment, the nephrotoxicity reducing agent of the present invention is used in an amount such that the weight ratio of the sugar is 30 or less, 25 or less, 20 or less, 15 or less, 13.3 or less, or 10 or less per 1 of the antisense oligomer.

In one embodiment, the nephrotoxicity reducing agent of the present invention is comprised in the pharmaceutical composition described herein and administered therewith. For example, the antisense oligomer described herein is optionally lyophilized with a carrier such as glucose, and then dissolved in a solvent such as water for injection to prepare a pharmaceutical composition. The resulting pharmaceutical composition may be mixed with the nephrotoxicity reducing agent described herein, and the amount of the mixture may then be optionally adjusted with a solvent to administer to a subject. Alternatively, for example, the antisense oligomer described herein is lyophilized with the nephrotoxicity reducing agent of the present invention, and then dissolved in a solvent such as water for injection to prepare a pharmaceutical composition. The amount thereof may then be optionally adjusted with a solvent to administer to a subject.

In another embodiment, the nephrotoxicity reducing agent of the present invention is not comprised in the pharmaceutical composition described herein, and is administered separately from (simultaneously or sequentially with) the pharmaceutical composition described herein. For example, a pharmaceutical composition obtained by dissolving a lyophilized agent of the antisense oligomer described herein in a solvent such as water for injection may be administered to a subject separately from the nephrotoxicity reducing agent described herein. As used herein, administering the nephrotoxicity reducing agent and the pharmaceutical composition "simultaneously" means administering the nephrotoxicity reducing agent and the pharmaceutical composition at the same time point. As used herein, administering the nephrotoxicity reducing agent and the pharmaceutical composition "sequentially" means administering each of them at a different time point. Specifically, the pharmaceutical composition can be administered before or after the nephrotoxicity reducing agent is administered, and in this case, a dosing interval between the nephrotoxicity reducing agent and the pharmaceutical composition is not limited, and may be, for example, several minutes, several hours, or up to about a day.

As used herein, examples of the subject to whom the pharmaceutical composition and/or nephrotoxicity reducing agent are administered include, but not limited to, mammals, such as primates such as humans, laboratory animals such as rats, mice, and Norway rats, and farm animals such as pigs, cattle, horses, and sheep, and preferred are humans.

The dose of the pharmaceutical composition and/or nephrotoxicity reducing agent when administered can be adjusted in consideration of the types of the antisense oligomer comprised in the pharmaceutical composition and the sugar comprised in the nephrotoxicity reducing agent, the dosage form of the pharmaceutical composition and nephrotoxicity reducing agent, the condition of the subject such as age or body weight, the administration route, and the nature and symptoms of the disease. The amount of the antisense oligomer can be in a range of 0.1 mg to 10 g/day/person, for example, in a range of 1 mg to 1 g/day/person, for example, or in a range of 10 mg to 100 mg/day/person, for example, and the amount of the sugar can be in a range of 0.1 mg to 200 g/day/person, for example, in a range of 1 mg to 100 g/day/person for example, in a range of 10 mg to 50 g/day/person, for example, in a range of 100 mg to 50 g/day/person, for example, in a range of 100 mg to 40 g/day/person, for example, in a range of 100 mg to 30 g/day/person, for example, in a range of 100 mg to 20 g/day/person, for example, in a range of 1 g to 20 g/day/person, for example, in a range of 2 g to 20 g/day/person, for example, in a range of 1 g to 10 g/day/person for example, or in a range of 100 mg to 1 g/day/person, for example. The number and frequency of administration are not limited, but for example, the administration can be performed once or two to three times a day at the interval of one day or two to three days. Further, for example, the administration can be performed only once, or another administration may be performed a few days later for a total of two administrations.

In one embodiment, the present invention relates to a method for reducing nephrotoxicity for a pharmaceutical composition comprising an antisense oligomer or a method for producing a pharmaceutical composition comprising an antisense oligomer with reduced nephrotoxicity, comprising adding a sugar in an amount such that the concentration of the sugar in the pharmaceutical composition is 1 mg/mL to 400 mg/mL. In the present embodiment, the antisense oligomer, the pharmaceutical composition, the sugar, the concentration of the sugar in the pharmaceutical composition and the like are as described herein.

In one embodiment, the present invention relates to a method for reducing nephrotoxicity of an antisense oligomer in a subject to whom the antisense oligomer or the pharmaceutical composition comprising the antisense oligomer has been administered, comprising administering a sugar or a nephrotoxicity reducing agent to the subject, wherein the sugar is administered in an amount such that the weight ratio of the sugar is 0.05 to 30 per 1 of the antisense oligomer. In the present embodiment, the antisense oligomer, the pharmaceutical composition, the sugar, the nephrotoxicity reducing agent, the weight ratio of the sugar per 1 of the antisense oligomer and the like are as described herein.

In one embodiment, the present invention relates to a pharmaceutical composition comprising an antisense oligomer with reduced nephrotoxicity, and comprising a nephrotoxicity reducing agent comprising a sugar at a concentration of 1 mg/mL to 400 mg/mL. In the present embodiment, the antisense oligomer, the pharmaceutical composition, the sugar, the concentration of the sugar in the pharmaceutical composition and the like are as described herein.

### Examples

### [Example 1: Production of Antisense Oligomer (PMO)]

The antisense oligomers (PMO Nos. 1 to 12 (SEQ ID NO: 1 to 12)) listed in Table 1 were synthesized according to the method described in International Publication No. WO2013/100190. The theoretical value and measured value measured by ESI-TOF-MS of the molecular weight of each antisense oligomer are also shown.

**[Table 1]**

| Table 1: Synthesized antisense oligomers (PMO No.1 to 12) | | | | | |
|---|---|---|---|---|---|
| PMO No. | Sequence | 5'-end | Molecular weight | | SEQ ID NO. |
| | | | Theoretical value | Measured value | |
| 1 | GCTCGGCATCAGAGGGAGACCG | Group (2) | 7353.55 | 7353.93 | 1 |
| 2 | CTCGGCATCAGAGGGAGACC | Group (2) | 6643.31 | 6643.91 | 2 |
| 3 | CGGCATCAGAGGGAGAC | Group (2) | 5682.99 | 5682.27 | 3 |
| 4 | CCTTGGCTCGGCATCAGAGG | Group (2) | 6625.29 | 6625.31 | 4 |
| 5 | CATCAGAGGGAGACCGTGTAA | Group (2) | 7021.45 | 7021.88 | 5 |
| 6 | CTCGTGCATCAGAGGGAGACC | Group (2) | 6973.42 | 6974.13 | 6 |
| 7 | CTCGCATCAGAGGGAGACC | Group (2) | 6288.19 | 6287.87 | 7 |
| 8 | AGGAGGGCAGCGAACTCCTGGTTGT | Group (2) | 8383.90 | 8383.85 | 8 |
| 9 | GTGGTTGAGCCAGAAGCTGGGAATT | Group (2) | 8422.91 | 8422.85 | 9 |
| 10 | GTTGCCTCCGGTTCTGAAGGTGTTC | Group (1) | 8643.00 | 8642.94 | 10 |
| 11 | CAATGCCATCCTGGAGTTCCTG | Group (1) | 7580.66 | 7580.17 | 11 |
| 12 | CCTCCGGTTCTGAAGGTGTTC | Group (2) | 6921.39 | 6921.66 | 12 |

"Group (1)" and "group (2)" in the table above are as follows.

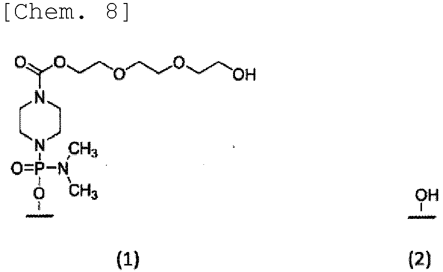

### [Example 2: Evaluation of Nephrotoxicity Reducing Effects of Sucrose]

Safety evaluation in the kidney was conducted on the antisense oligomers of PMO Nos. 8 and 9 (SEQ ID NO: 8 and 9) among those listed in Table 1 to verify their utility for pharmaceutical applications.

### (1) Evaluation Method

The antisense oligomers of PMO Nos. 8 and 9 were each dissolved in water for injection comprising 0.9% sodium chloride to prepare a control dosing solution comprising each of the antisense oligomers at the concentrations shown in Tables 2 and 3 and comprising no sucrose.

Next, the antisense oligomers of PMO Nos. 8 and 9 were each dissolved in water for injection comprising 0.9% sodium chloride, and a solution obtained by dissolving sucrose (manufactured by FUJIFILM Wako Pure Chemical Corporation) with physiological saline was added thereto to prepare a dosing solution comprising each of the antisense oligomers and sucrose at the concentrations shown in Tables 2 and 3.

The obtained dosing solutions were administered into the tail vein of 6-week-old C57BL/6J male mice (N=3) at 10 and 12 mL/kg. Next day, serum was collected from the mice, and the blood urea nitrogen (BUN) values and blood creatinine (Cre) values were measured with the urease-GIDH method and the enzymatic method, respectively, using an automatic biochemical analyzer JCA-BM8060 (manufactured by JEOL Ltd.). Further, some mice to which the antisense oligomer of PMO No. 8 had been administered were subjected to autopsy, and formalin-fixed paraffin-embedded samples of the respective kidneys were prepared. Sections obtained by slicing the samples were subjected to hematoxylin-eosin staining and a histopathological test of the sections was performed. It was determined that the nephrotoxicity was reduced in a case where both of the BUN values and Cre values decreased in the mice to which the dosing solution comprising sucrose has been administered, compared to the mice to which the control dosing solution comprising no sucrose has been administered.

### (2) Evaluation Results

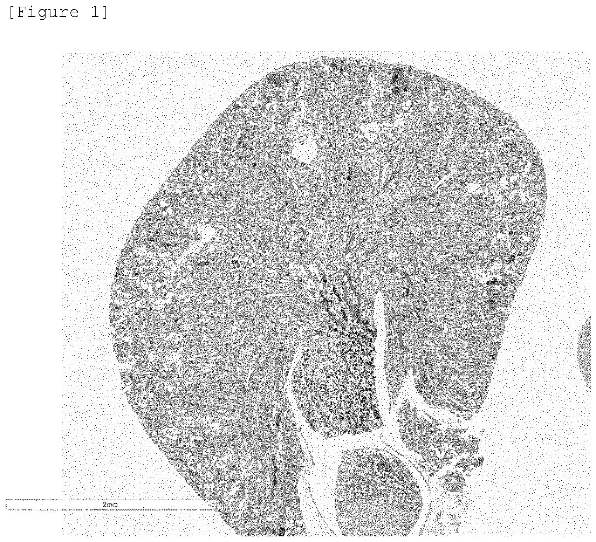

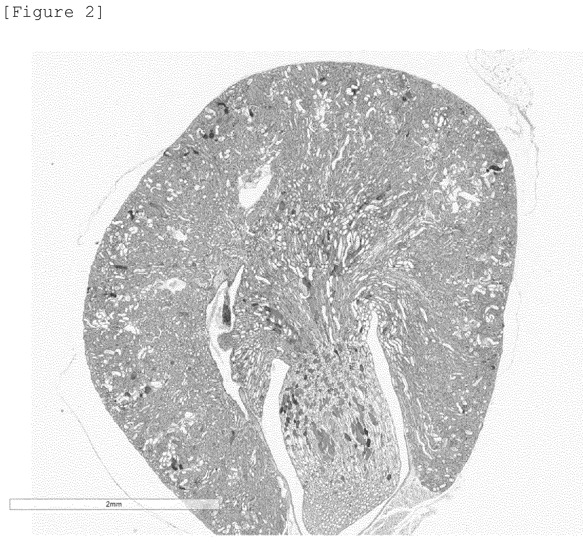

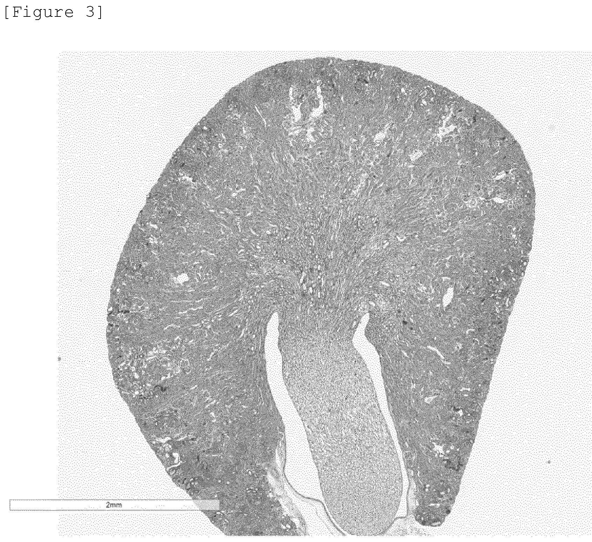

Since each of the antisense oligomers tested was confirmed that both of the BUN values and Cre values were decreased in the mice to which the dosing solution comprising sucrose has been administered, sucrose was confirmed to reduce nephrotoxicity. Also, the histopathological test revealed that basophilic substances were observed in the lumens of renal tubules in the kidneys of the mice to which the control dosing solution comprising no sucrose has been administered. This change was thought to be caused by precipitation of nucleic acids. The amount of precipitates decreased according to the concentrations of sucrose, which coincided with the nephrotoxicity reducing effects. The results are shown in Tables 2, 3, and 4, and Figures 1, 2, 3, and 4.

**[Table 2]**

| Table 2: BUN values and Cre values when administering antisense oligomer (PMO No. 8) | | | | | | | |
|---|---|---|---|---|---|---|---|
| Amount of dosing solution (mL/kg) | Concentration of PMO (mg/mL) | Concentration of sucrose (mg/mL) | Weight ratio of sucrose /PMO | BUN (mg/dL) | Standard deviation of BUN (mg/dL) | Cre (mg/dL) | Standard deviation of Cre (mg/dL) |
| - | - | - | - | 34.7 | 1.7 | 0.13 | 0.01 |
| 10 | 50 | 0 | 0 | 253.1 | 10.2 | 1.81 | 0.05 |
| 10 | 50 | 5 | 0.1 | 238.0 | 8.8 | 1.68 | 0.22 |
| 10 | 50 | 10 | 0.2 | 255.4 | 37.6 | 1.26 | 0.49 |
| 10 | 50 | 20 | 0.4 | 217.5 | 32.5 | 0.90 | 0.38 |
| 10 | 50 | 50 | 1 | 64.0 | 9.0 | 0.16 | 0.01 |
| 10 | 50 | 100 | 2 | 33.8 | 1.5 | 0.10 | 0.01 |

| | | | | | | | |
|---|---|---|---|---|---|---|---|
| - : No administration | | | | | | | |

**[Table 3]**

| Table 3: BUN values and Cre values when administering antisense oligomer (PMO No. 9) | | | | | | | |
|---|---|---|---|---|---|---|---|
| Amount of dosing solution (mL/kg) | Concentration of PMO (mg/mL) | Concentration of sucrose (mg/mL) | Weight ratio of sucrose /PMO | BUN (mg/dL) | Standard: deviation of BUN (mg/dL) | Cre (mg/dL) | Standard deviation of Cre (mg/dL) |
| - | - | - | - | 29.8 | 3.6 | 0.11 | 0.02 |
| 12 | 16.667 | 0 | 0 | 186.6 | 59.8 | 0.80 | 0.57 |
| 12 | 16. 667 | 16.667 | 1 | 158.1 | 87.3 | 0.96 | 0.56 |
| 12 | 16.667 | 41.667 | 2.5 | 75.2 | 75.2 | 0.30 | 0.30 |
| 12 | 16.667 | 83.333 | 5 | 30.4 | 3.2 | 0.13 | 0.04 |
| 12 | 16.667 | 166.667 | 10 | 29.8 | 2.4 | 0.10 | 0.01 |
| 12 | 25 | 0 | 0 | 192.9 | 58.4 | 0.86 | 0.46 |
| 12 | 25 | 83.333 | 3.333 | 31.0 | 2.0 | 0.10 | 0.02 |
| 12 | 25 | 166.667 | 6.667 | 28.3 | 3.6 | 0.11 | 0.02 |
| 12 | 25 | 333.333 | 13.333 | 31.8 | 2.4 | 0.12 | 0.01 |

| | | | | | | | |
|---|---|---|---|---|---|---|---|
| - : No administration | | | | | | | |

**[Table 4]**

| Table 4: Histopathological test of kidney when administering antisense oligomer (PMO No. 8) | | | | | | |
|---|---|---|---|---|---|---|
| Amount of dosing solution (mL/kg) | Concentration of PMO (mg/mL) | Concentration of sucrose (mg/mL) | Weight ratio of sucrose /PMO | Basophilic substances in renal tubules | Expansion of renal tubules | Renal tubular necrosis in inner stripe of outer medulla |
| - | - | - | - | - | - | - |
| 10 | 50 | 0 | 0 | 2+ (3/3) | 3+ (3/3) | ± (3/3) |
| 10 | 50 | 20 | 0.4 | 2+ (3/3) | 3+ (3/3) | 1+ (1/3) |
| | | | | | | ± (2/3) |
| 10 | 50 | 50 | 1 | 1+ (3/3) | 2+ (1/3) | ± (1/3) |
| | | | | | 1+ (2/3) | |
| 10 | 50 | 100 | 2 | ± (3/3) | - | - |

| | | | | | | |
|---|---|---|---|---|---|---|
| -: Not changed ±: The case where the change is very slight and thus the organ/tissue is considered to function normally. 1+: The case where the change is definite but its distribution or degree is limited, and thus the organ/tissue is considered to be rarely affected functionally. 2+: The case where the change is definite and observed widely, and thus the organ/tissue is considered to be affected to some extent functionally. 3+: The case where the change is significant and thus the organ/tissue is considered to be definitely affected functionally. | | | | | | |

The above results showed that sucrose reduces the nephrotoxicity due to the administration of antisense oligomers.

### [Example 3: Evaluation of Nephrotoxicity Reducing Effects of Sucrose]

Safety evaluation in the kidney was conducted on the antisense oligomers of PMO No. 11 (SEQ ID NO: 11) among those listed in Table 1 to verify their utility for pharmaceutical applications.

### (1) Evaluation Method

The antisense oligomer of PMO No. 11 was dissolved in physiological saline to prepare a control dosing solution comprising the antisense oligomer at the concentration shown in Table 5 and comprising no sucrose.

Next, the antisense oligomer of PMO No. 11 and sucrose were dissolved in physiological saline to prepare dosing solutions comprising the antisense oligomer and sucrose at the concentrations shown in Table 5.

The obtained dosing solutions were administered into the tail vein of 6-week-old C57BL/6J male mice (N=5) at 20 mL/kg. Next day, the mice were subjected to autopsy, and formalin-fixed paraffin-embedded samples of the respective kidneys were prepared. Sections obtained by slicing the samples were subjected to hematoxylin-eosin staining and a histopathological test of the sections was performed. It was determined that the nephrotoxicity was reduced in a case where improvement in the toxicological findings was observed on the histopathological test of the kidney of the mice to which the dosing solution comprising sucrose has been administered, compared to the mice to which the dosing solution comprising no sucrose has been administered.

### (2) Evaluation Results

Since the antisense oligomer tested was confirmed that improvement in the toxicological findings was observed on the histopathological test of the kidney of the mice to which the dosing solution comprising sucrose has been administered, sucrose was confirmed to reduce nephrotoxicity. The results are shown in Table 5.

**[Table 5]**

| Table 5: Toxicological Findings of Histopathological test of kidney when administering antisense oligomer (PMO No. 11) | | | | | |
|---|---|---|---|---|---|
| Amount of dosing solution (mL/kg) | Concentration of PMO (mg/mL) | Concentration of sucrose (mg/mL) | Weight ratio of sucrose /PMO | Renal tubular necrosis in outer stripe of outer medulla | Hemorrhage /congestion in inner stripe of outer medulla |
| 20* | 0 | 0 | 0 | - | - |
| 20 | 130 | 0 | 0 | ± (3/5) | ± (2/5) |
| 20 | 130 | 200 | 1. 538 | ± (1/5) | - |

| | | | | | |
|---|---|---|---|---|---|
| *: Physiological saline administered -: Not changed ±: The case where the change is very slight and thus the organ/tissue is considered to function normally. | | | | | |

The above results showed that sucrose reduces the nephrotoxicity due to the administration of antisense oligomers.

### [Example 4: Evaluation of Precipitation Suppressing Effects with Sucrose]

The amount of precipitation in the presence of ions assumed to be in urine and precipitation suppressing effects of sucrose were evaluated on the antisense oligomers of PMO Nos. 8, 9, 10, and 11 among those listed in Table 1 to further verify their utility for pharmaceutical applications. If suppressed precipitation results in the reduced accumulation of the antisense oligomer in the kidney in vivo, it is expected to lead to reduced nephrotoxicity.

### Test Method

Each of the antisense oligomers was dissolved in water for injection or an aqueous solution of sucrose, and the mixture was then mixed with an aqueous solution comprising potassium chloride and sodium chloride assumed to be in urine. Evaluation conditions (solution compositions) are shown in Tables 6 to 13. The absorbance at 620 nm of the solution thus mixed was measured using a plate reader, Infinite F200 Pro (manufactured by Tecan Group Ltd.) under heating conditions of 37°C. Assuming the precipitation of antisense oligomers in urine, the concentrations of the antisense oligomers, potassium chloride and sodium chloride vary depending on the amount of the antisense oligomer administered and/or the amount of urine. Accordingly, conditions were set under which the precipitation was observed using water for injection as a medium for each sequence. Measurement was performed under the same conditions as obtained except that only the medium was changed to an aqueous solution of sucrose, and then the absorbance obtained under both the conditions was compared to each other. When the absorbance was increased, the precipitation was determined to be observed, and a time until the precipitation was observed and the increased value of the absorbance were evaluated. When the aqueous solution of sucrose was used as the medium, it was determined that the precipitation suppressing effect was presented in a case where the time until the precipitation was observed was longer or in a case where the increased value of the absorbance was smaller than the case of using the water for injection as the medium.

**[Table 6]**

| Table 6: Evaluation conditions of precipitation suppression by sucrose in PMO No. 8 | | | | |
|---|---|---|---|---|
| PMO No. | PMO (mg/mL) | KCl (mmol/mL ) | NaCl (mmol/mL ) | Sucrose (mg/mL) |
| 8 | 16.5 | 100 | 77 | 0 |
| 8 | 16.5 | 100 | 77 | 135 |
| 8 | 16.5 | 100 | 77 | 270 |

**[Table 7]**

| Table 7: Evaluation conditions of precipitation suppression by sucrose in PMO No. 8 | | | | |
|---|---|---|---|---|
| PMO No. | PMO (mg/mL) | KCl (mmol/mL ) | NaCl (mmol/mL ) | Sucrose (mg/mL) |
| 8 | 14.667 | 200 | 154 | 0 |
| 8 | 14.667 | 200 | 154 | 120 |

**[Table 8]**

| Table 8: Evaluation conditions of precipitation suppression by sucrose in PMO No. 8 | | | | |
|---|---|---|---|---|
| PMO No. | PMO (mg/mL) | KCl (mmol/mL ) | NaCl (mmol/mL ) | Sucrose (mg/mL) |
| 8 | 16.5 | 100 | 77 | 0 |
| 8 | 16.5 | 100 | 77 | 90 |
| 8 | 16.5 | 100 | 77 | 72 |
| 8 | 16.5 | 100 | 77 | 45 |

**[Table 9]**

| Table 9: Evaluation conditions of precipitation suppression by sucrose in PMO No. 9 | | | | |
|---|---|---|---|---|
| PMO No. | PMO (mg/mL) | KCl (mmol/mL ) | NaCl (mmol/mL ) | Sucrose (mg/mL) |
| 9 | 12.375 | 100 | 77 | 0 |
| 9 | 12.375 | 100 | 77 | 135 |
| 9 | 12.375 | 100 | 77 | 270 |

**[Table 10]**

| Table 10: Evaluation conditions of precipitation suppression by sucrose in PMO No. 9 | | | | |
|---|---|---|---|---|
| PMO No. | PMO (mg/mL) | KCl (mmol/mL ) | NaCl (mmol/mL ) | Sucrose (mg/mL) |
| 9 | 12.375 | 200 | 154 | 0 |
| 9 | 12.375 | 200 | 154 | 120 |

**[Table 11]**

| Table 11: Evaluation conditions of precipitation suppression by sucrose in PMO No. 9 | | | | |
|---|---|---|---|---|
| PMO No. | PMO (mg/mL) | KCl (mmol/mL ) | NaCl (mmol/mL ) | Sucrose (mg/mL) |
| 9 | 12.375 | 100 | 77 | 0 |
| 9 | 12.375 | 100 | 77 | 90 |
| 9 | 12.375 | 100 | 77 | 72 |
| 9 | 12.375 | 100 | 77 | 45 |

**[Table 12]**

| Table 12: Evaluation conditions of precipitation suppression by sucrose in PMO No. 10 | | | | |
|---|---|---|---|---|
| PMO No. | PMO (mg/mL) | KCl (mmol/mL ) | NaCl (mmol/mL ) | Sucrose (mg/mL) |
| 10 | 110 | 1500 | 385 | 0 |
| 10 | 110 | 1500 | 385 | 75 |
| 10 | 110 | 1500 | 385 | 120 |

**[Table 13]**

| Table 13: Evaluation conditions of precipitation suppression by sucrose in PMO No. 11 | | | | |
|---|---|---|---|---|
| PMO No. | PMO (mg/mL) | KCl (mmol/mL ) | NaCl (mmol/mL ) | Sucrose (mg/mL) |
| 11 | 110 | 1800 | 462 | 0 |
| 11 | 110 | 1800 | 462 | 120 |
| 11 | 110 | 1800 | 462 | 60 |

### Test Results

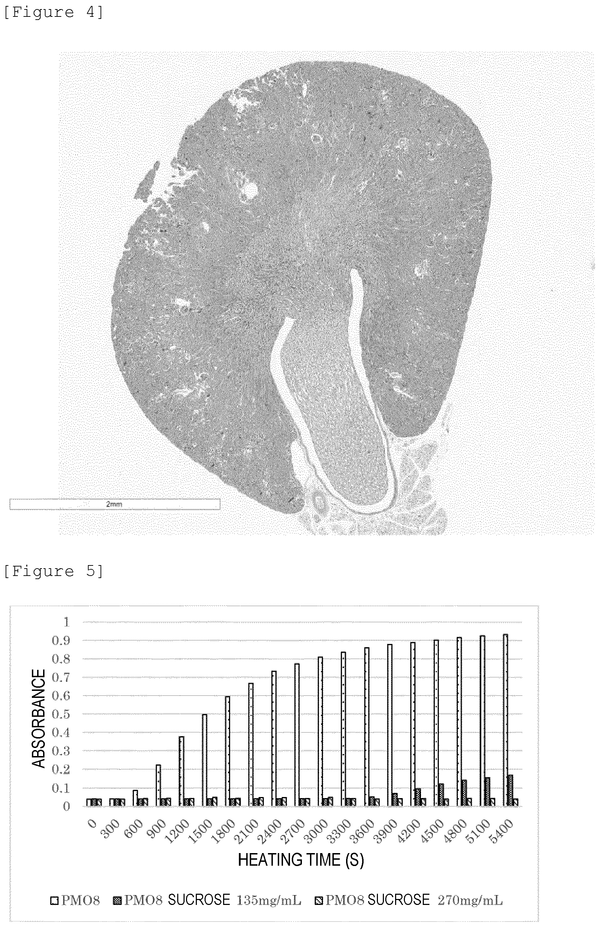

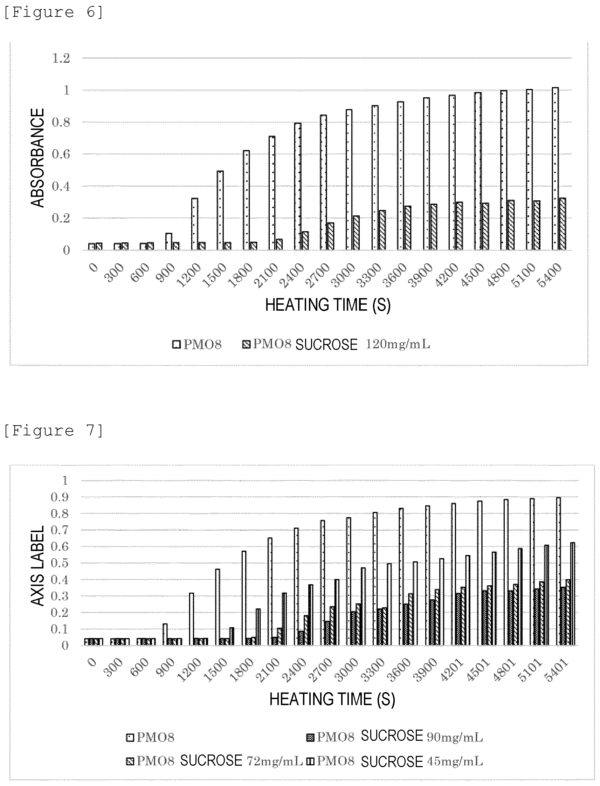

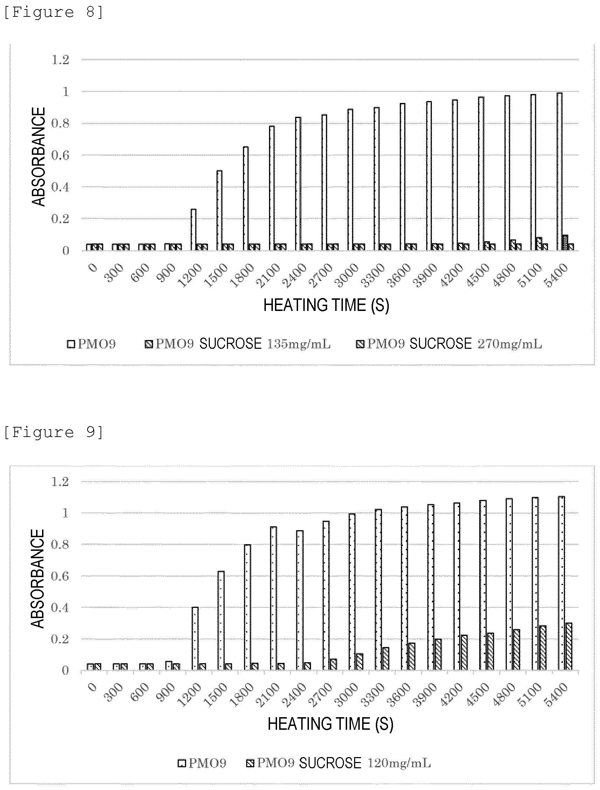

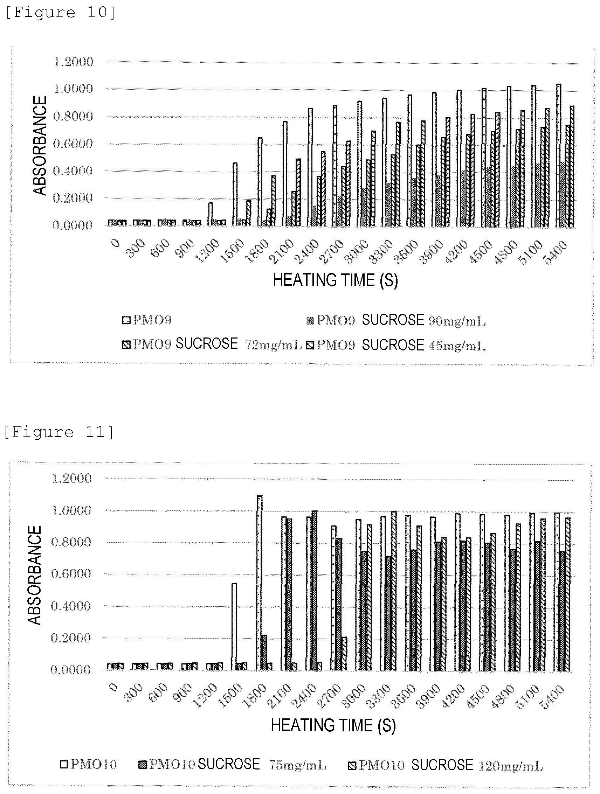

Since each of the antisense oligomers tested prolonged the time until the precipitation was observed and suppressed the increase of the absorbance in all the cases of using the aqueous solution of sucrose as the medium, sucrose was indicated to suppress the precipitation of antisense oligomers. The respective results are shown in Figures 5 to 12.

The above results showed that sucrose suppresses the precipitation of the antisense oligomers in urine.

### [Example 5: Evaluation of Nephrotoxicity Reducing Effects with Sucrose and Trehalose]

Safety evaluation in the kidney was conducted on the antisense oligomers of PMO Nos. 1, 8, and 9 (SEQ ID NO: 1, 8, and 9) among those listed in Table 1 to verify their utility for pharmaceutical applications.

### (1) Evaluation Method

The antisense oligomers of PMO Nos. 1, 8 and 9 were each dissolved in water for injection comprising 0.9% sodium chloride to prepare a control dosing solution comprising each of the antisense oligomers at the concentrations shown in Tables 14 to 17 and comprising no sucrose nor trehalose.

Next, the antisense oligomer of PMO No. 1 was dissolved in water for injection comprising 0.9% sodium chloride, and a solution obtained by dissolving sucrose (manufactured by FUJIFILM Wako Pure Chemical Corporation) with physiological saline was added thereto to prepare a dosing solution each comprising the antisense oligomer and sucrose at the concentrations shown in Table 14. Also, the antisense oligomers of PMO Nos. 1, 8, and 9 were each dissolved in water for injection comprising 0.9% sodium chloride, and a solution obtained by dissolving trehalose (manufactured by FUJIFILM Wako Pure Chemical Corporation) with physiological saline was added thereto to prepare a dosing solution comprising each of the antisense oligomers and trehalose at the concentrations shown in Tables 15 to 17.

The obtained dosing solutions were administered into the tail vein of 6-week-old C57BL/6J male mice (N=2 to 3) at 20 mL/kg. Next day, serum was collected from the mice, and the blood urea nitrogen (BUN) values and blood creatinine (Cre) values were measured with the urease-GIDH method and the enzymatic method, respectively, using an automatic biochemical analyzer JCA-BM8060 (manufactured by JEOL Ltd.). It was determined that the nephrotoxicity was reduced in a case where both of the BUN values and Cre values decreased in the mice to which the dosing solution comprising sucrose or trehalose has been administered, compared to the mice to which the dosing solution comprising neither sucrose nor trehalose has been administered.

### (2) Evaluation Results

Since each of the antisense oligomers tested was confirmed that both of the BUN values and Cre values were decreased in the mice to which the dosing solution comprising sucrose or trehalose has been administered, sucrose and trehalose were confirmed to reduce nephrotoxicity. The results are shown in Tables 14 to 17.

**[Table 14]**

| Table 14: BUN values and Cre values when administering antisense oligomer (PMO No. 1) | | | | | | | |
|---|---|---|---|---|---|---|---|
| Amount of dosing solution (mL/kg) | Concentration of PMO (mg/mL) | Concentration of sucrose (mg/mL) | Weight ratio of sucrose /PMO | BUN (mg/dL) | Standard deviation of BUN (mg/dL) | Cre (mg/dL) | Standard deviation of Cre (mg/dL) |
| - | - | - | - | 29.4 | 1.7 | 0.1 | 0.02 |
| 20 | 300 | 0 | 0 | 221. 8 | 122.3 | 0.91 | 0.69 |
| 20 | 300 | 300 | 1 | 32.5 | 7.4 | 0.11 | 0.03 |
| 20 | 300 | 1000 | 3.333 | 28.3 | 1.2 | 0.09 | 0.02 |

| | | | | | | | |
|---|---|---|---|---|---|---|---|
| - : No administration | | | | | | | |

**[Table 15]**

| Table 15: BUN values and Cre values when administering antisense oligomer (PMO No. 1) | | | | | | | |
|---|---|---|---|---|---|---|---|
| Amount of dosing solution (mL/kg) | Concentration of PMO (mg/mL) | Concentration of trehalose (mg/mL) | Weight ratio of trehalose /PMO | BUN (mg/dL) | Standard deviation of BUN (mg/dL) | Cre (mg/dL) | Standard deviation of Cre (mg/dL) |
| - | - | - | - | 29. 4 | 1. 7 | 0. 1 | 0.02 |
| 20 | 300 | 0 | 0 | 221. 8 | 122.3 | 0.91 | 0.69 |
| 20 | 300 | 300 | 1 | 32.5 | 7.4 | 0.11 | 0.03 |
| 20 | 300 | 1000 | 3.333 | 28.3 | 1.2 | 0.09 | 0.02 |
| 20 | 200 | 1500 | 5 | 28. 3 | 1.2 | 0.09 | 0.02 |

| | | | | | | | |
|---|---|---|---|---|---|---|---|
| - : No administration | | | | | | | |

**[Table 16]**

| Table 16: BUN values and Cre values when administering antisense oligomer (PMO No. 8) | | | | | | | |
|---|---|---|---|---|---|---|---|
| Amount of dosing solution (mL/kg) | Concentration of PMO (mg/mL) | Concentration of trehalose (mg/mL) | Weight ratio of trehalose /PMO | BUN (mg/dL) | Standard deviation of BUN (mg/dL) | Cre (mg/dL) | Standard deviation of Cre (mg/dL) |
| - | - | - | - | 26. 6 | 0.8 | 0.11 | 0.01 |
| 20 | 500 | 0 | 0 | 251.4 | 14.6 | 2.39 | 0.17 |
| 20 | 500 | 2000 | 4 | 25.8 | 4.1 | 0.13 | 0.03 |
| 20 | 500 | 4000 | 8 | 25.9 | 2.5 | 0. 09 | 0. 02 |

| | | | | | | | |
|---|---|---|---|---|---|---|---|
| - : No administration | | | | | | | |

**[Table 17]**

| Table 17: BUN values and Cre values when administering antisense oligomer (PMO No. 9) | | | | | | | |
|---|---|---|---|---|---|---|---|
| Amount of dosing solution (mL/kg) | Concentration of PMO (mg/mL) | Concentration of trehalose (mg/mL) | Weight ratio of trehalose /PMO | BUN (mg/dL) | Standard deviation of BUN (mg/dL) | Cre (mg/dL) | Standard deviation of Cre (mg/dL) |
| - | - | - | - | 26.6 | 0.8 | 0.11 | 0.01 |
| 20 | 200 | 0 | 0 | 182. 5 | 43.8 | -3.64 | 0.33 |
| 20 | 200 | 300 | 1.5 | 25. 6 | 1.5 | 0.10 | 0.03 |
| 20 | 200 | 1000 | 5 | 25. 1 | 1.0 | 3.12 | 0.01 |

| | | | | | | | |
|---|---|---|---|---|---|---|---|
| - : No administration | | | | | | | |

### [Example 6: Evaluation of Precipitation Suppressing Effects with Sucrose]

The amount of precipitation in the presence of ions assumed to be in urine and precipitation suppressing effects of sucrose were evaluated on the antisense oligomers of PMO Nos. 1, 2, and 7 among those listed in Table 1 to further verify their utility for pharmaceutical applications. If suppressed precipitation results in the reduced accumulation of the antisense oligomer in the kidney in vivo, it is expected to lead to reduced nephrotoxicity.

### Test Method

Each of the antisense oligomers was dissolved in water for injection or an aqueous solution of sucrose, and the mixture was then mixed with an aqueous solution comprising potassium chloride and sodium chloride assumed to be in urine. Evaluation conditions (solution compositions) are shown in Tables 18 to 20. The absorbance at 620 nm of the solution thus mixed was measured using a plate reader, Infinite F200 Pro (manufactured by Tecan Group Ltd.) under the heating condition of 37°C. Assuming the precipitation of antisense oligomers in urine, the concentrations of the antisense oligomers, potassium chloride and sodium chloride vary depending on the amount of the antisense oligomer administered and/or the amount of urine. Accordingly, conditions were set under which the precipitation was observed using water for injection as a medium for each sequence. Another measurement was performed under the same conditions as obtained except that only the medium was changed to an aqueous solution of sucrose, and then the absorbance obtained under both the conditions was compared to each other. When the absorbance was increased, precipitation was determined to be observed, and a time until the precipitation was observed and the increased value of the absorbance were evaluated. When the aqueous solution of sucrose was used as the medium, it was determined that the precipitation suppressing effect was presented in a case where the time until the precipitation was observed was longer or in a case where the increased value of the absorbance was smaller than the case of using the water for injection as the medium.

**[Table 18]**

| Table 18: Evaluation conditions of precipitation suppression by sucrose in PMO No. 1 | | | | |
|---|---|---|---|---|
| PMO No. | PMO (mg/mL) | KCl (mmol/mL) | NaCl (mmol/mL) | Sucrose (mg/mL) |
| 1 | 24.75 | 100 | 77 | 0 |
| 1 | 24.75 | 100 | 77 | 135 |
| 1 | 24. 75 | 100 | 77 | 45 |

**[Table 19]**

| Table 19: Evaluation conditions of precipitation suppression by sucrose in PMO No. 2 | | | | |
|---|---|---|---|---|
| PMO No. | PMO (mg/mL) | KCl (mmol/mL) | NaCl (mmol/mL) | Sucrose (mg/mL) |
| 2 | 49. 5 | 100 | 77 | 0 |
| 2 | 49.5 | 100 | 77 | 135 |
| 2 | 49.5 | 100 | 77 | 45 |

**[Table 20]**

| Table 20: Evaluation conditions of precipitation suppression by sucrose in PMO No. 7 | | | | |
|---|---|---|---|---|
| PMO No. | PMO (mg/mL) | KCl (mmol/mL) | NaCl (mmol/mL) | Sucrose (mg/mL) |
| 7 | 264 | 400 | 154 | 0 |
| 7 | 264 | 400 | 154 | 120 |
| 7 | 264 | 400 | 154 | 80 |

### Test Results

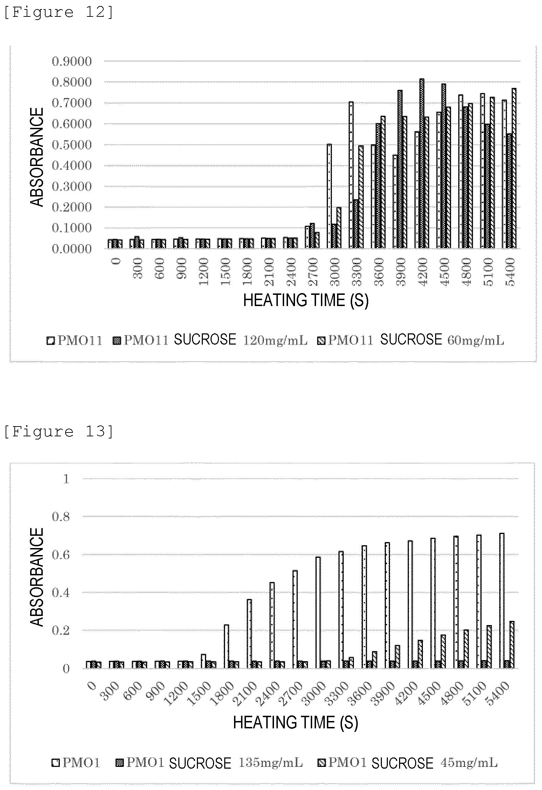

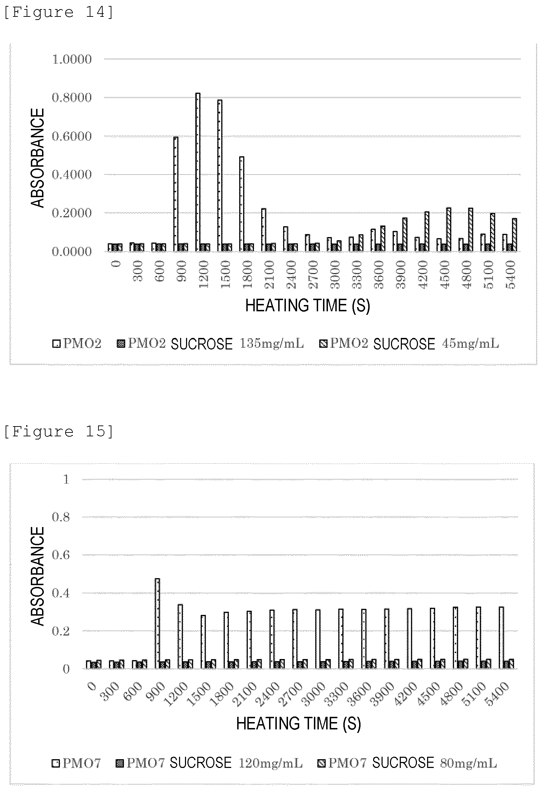

Since each of the antisense oligomers tested prolonged the time until the precipitation was observed and suppressed the increase of the absorbance in all the cases of using the aqueous solution of sucrose as the medium, sucrose was indicated to suppress the precipitation of antisense oligomers. The respective results are shown in Figures 13 to 15.

The above results showed that sucrose suppresses the precipitation of the antisense oligomers in urine.

### [Example 7: Evaluation of Precipitation Suppressing Effects with Trehalose]

The amount of precipitation in the presence of ions assumed to be in urine and precipitation suppressing effects of trehalose were evaluated on the antisense oligomers of PMO Nos. 1, 2, 7, and 8 among those listed in Table 1 to further verify their utility for pharmaceutical applications. If suppressed precipitation results in the reduced accumulation of the antisense oligomer in the kidney in vivo, it is expected to lead to reduced nephrotoxicity.

### Test Method

Each of the antisense oligomers was dissolved in water for injection or an aqueous solution of trehalose, and the mixture was then mixed with an aqueous solution comprising potassium chloride and sodium chloride assumed to be in urine. Evaluation conditions (solution compositions) are shown in Tables 21 to 24. The absorbance at 620 nm of the solution thus mixed was measured using a plate reader, Infinite F200 Pro (manufactured by Tecan Group Ltd.) under the heating condition of 37°C. Assuming the precipitation of antisense oligomers in urine, the concentrations of the antisense oligomers, potassium chloride and sodium chloride vary depending on the amount of the antisense oligomer administered and/or the amount of urine. Accordingly, conditions were set under which the precipitation was observed using water for injection as a medium for each sequence. Another measurement was performed under the same conditions as obtained except that only the medium was changed to an aqueous solution of trehalose, and then the absorbance obtained under both the conditions was compared to each other. When the absorbance was increased, precipitation was determined to be observed, and a time until the precipitation was observed and the increased value of the absorbance were evaluated. When the aqueous solution of trehalose was used as the medium, it was determined that the precipitation suppressing effect was presented in a case where the time until the precipitation was observed was longer or in a case where the increased value of the absorbance was smaller than the case of using the water for injection as the medium.

**[Table 21]**

| Table 21: Evaluation conditions of precipitation suppression by trehalose in PMO No. 1 | | | | |
|---|---|---|---|---|
| PMO No. | PMO (mg/mL) | KCl (mmol/mL) | NaCl (mmol/mL) | Trehalose (mg/mL) |
| 1 | 24. 75 | 100 | 77 | 0 |
| 1 | 24. 75 | 100 | 77 | 45 |
| 1 | 24. 75 | 100 | 77 | 90 |
| 1 | 24. 75 | 100 | 77 | 135 |

**[Table 22]**

| Table 22: Evaluation conditions of precipitation suppression by trehalose in PMO No. 2 | | | | |
|---|---|---|---|---|
| PMO No. | PMO (mg/mL) | KCl (mmol/mL) | NaCl (mmol/mL) | Trehalose (mg/mL) |
| 2 | 49. 5 | 100 | 77 | 0 |
| 2 | 49. 5 | 100 | 77 | 45 |

**[Table 23]**

| Table 23: Evaluation conditions of precipitation suppression by trehalose in PMO No. 7 | | | | |
|---|---|---|---|---|
| PMO No. | PMO (mg/mL) | KCl (mmol/mL) | NaCl (mmol/mL) | Trehalose (mg/mL) |
| 7 | 264 | 400 | 154 | 0 |
| 7 | 264 | 400 | 154 | 80 |

**[Table 24]**

| Table 24: Evaluation conditions of precipitation suppression by trehalose in PMO No. 8 | | | | |
|---|---|---|---|---|
| PMO No. | PMO (mg/mL) | KCl (mmol/mL) | NaCl (mmol/mL) | Trehalose (mg/mL) |
| 8 | 16. 5 | 100 | 77 | 0 |
| 8 | 16. 5 | 100 | 77 | 45 |
| 8 | 16. 5 | 100 | 77 | 90 |
| 8 | 16. 5 | 100 | 77 | 135 |

### Test Results

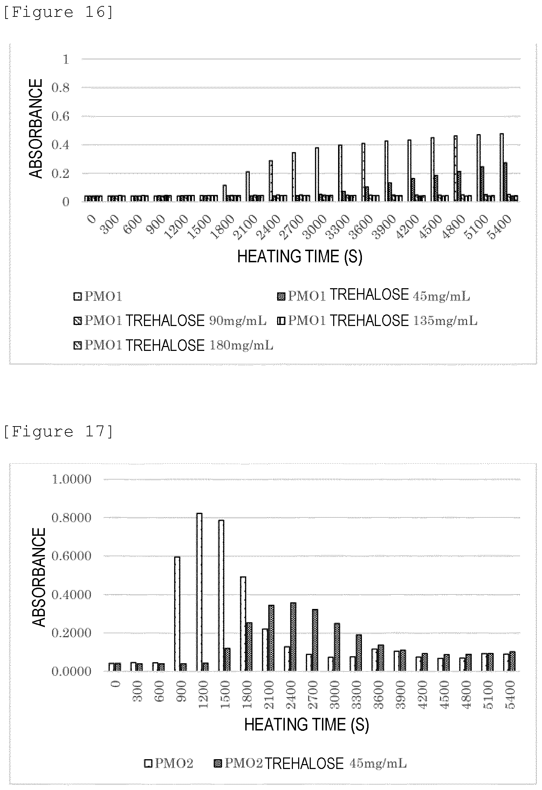

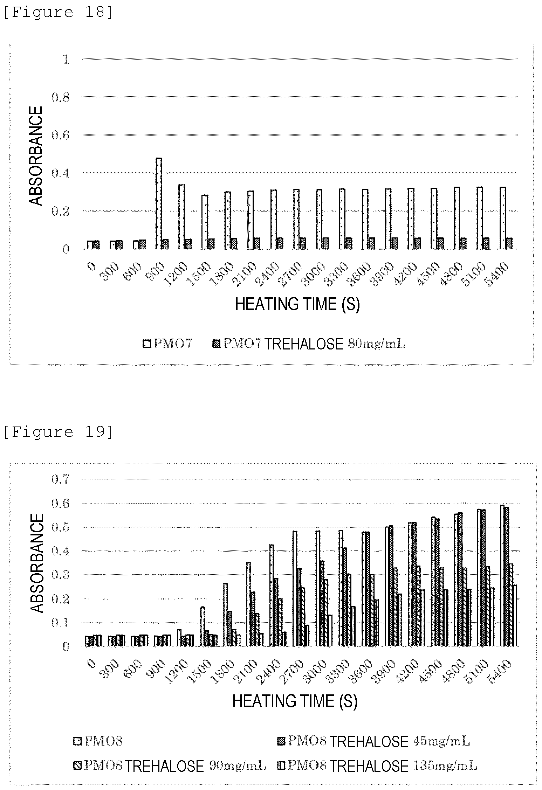

Since each of the antisense oligomers tested prolonged the time until the precipitation was observed and suppressed the increase of the absorbance in all the cases of using the aqueous solution of trehalose as the medium, trehalose was indicated to suppress the precipitation of antisense oligomers. The respective results are shown in Figures 16 to 19.

The above results showed that trehalose suppresses the precipitation of the antisense oligomers in urine.

## Claims

1. A nephrotoxicity reducing agent for a pharmaceutical composition comprising an antisense oligomer, wherein the nephrotoxicity reducing agent comprises a non-glucose sugar and is used in an amount such that a concentration of the sugar in the pharmaceutical composition is 1 mg/mL to 400 mg/mL.

2. The nephrotoxicity reducing agent according to claim 1, wherein the nephrotoxicity reducing agent is used in an amount such that the concentration of the sugar in the pharmaceutical composition is 5 mg/mL to 340 mg/mL.

3. The nephrotoxicity reducing agent according to claim 1 or 2, wherein a concentration of the antisense oligomer in the pharmaceutical composition is 0.5 mg/mL to 200 mg/mL.

4. A nephrotoxicity reducing agent for a pharmaceutical composition comprising an antisense oligomer, wherein the nephrotoxicity reducing agent comprises a non-glucose sugar and is used in an amount such that a weight ratio of the sugar is 0.05 to 30 per 1 of the antisense oligomer.

5. The nephrotoxicity reducing agent according to claim 4, wherein the nephrotoxicity reducing agent is used in an amount such that the weight ratio of the sugar is 0.1 to 13.3 per 1 of the antisense oligomer.

6. The nephrotoxicity reducing agent according to any one of claims 1 to 5, wherein the sugar is a disaccharide.

7. The nephrotoxicity reducing agent according to any one of claims 1 to 6, wherein the sugar is sucrose.

8. The nephrotoxicity reducing agent according to any one of claims 1 to 6, wherein the sugar is trehalose.

9. The nephrotoxicity reducing agent according to any one of claims 1 to 8, wherein the antisense oligomer is a morpholino oligomer.

10. The nephrotoxicity reducing agent according to any one of claims 1 to 9, wherein the antisense oligomer is a phosphorodiamidate morpholino oligomer.

11. The nephrotoxicity reducing agent according to any one of claims 1 to 10, wherein the 5'-end of the antisense oligomer is any group represented by the following chemical formulae (1) and (2):

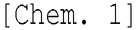

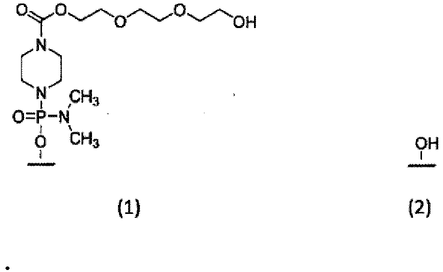

12. The nephrotoxicity reducing agent according to any one of claims 1 to 11, wherein the antisense oligomer comprises four consecutive purine bases in its base sequence.

13. The nephrotoxicity reducing agent according to claim 12, wherein at least two of the four consecutive purine bases are guanine.

14. The nephrotoxicity reducing agent according to any one of claims 1 to 13, wherein the antisense oligomer comprises a base sequence selected from the group consisting of SEQ ID NOs: 1 to 12.

15. A method for reducing nephrotoxicity for a pharmaceutical composition comprising an antisense oligomer, comprising adding a non-glucose sugar in an amount such that a concentration of the sugar in the pharmaceutical composition is 1 mg/mL to 400 mg/mL.

16. A method for reducing nephrotoxicity of an antisense oligomer in a subject to whom the antisense oligomer has been administered, comprising administering a non-glucose sugar to the subject, wherein the sugar is administered in an amount such that a weight ratio of the sugar is 0.05 to 30 per 1 of the antisense oligomer.

17. A pharmaceutical composition comprising an antisense oligomer and having reduced nephrotoxicity, wherein the pharmaceutical composition comprises a nephrotoxicity reducing agent comprising a non-glucose sugar at a concentration of 1 mg/mL to 400 mg/mL.
